# DEMANDE DE BREVET EUROPEEN

(11) **EP 4 582 631 A1**
(43) Date de publication de la demande: **09.07.2025**
(21) Numéro de dépôt: 24219805.9
(22) Date de dépôt: 13.12.2024
(51) Int. Cl.: D06N 3/00, A61F 13/02, C08J 9/36, C08J 9/42, D06N 3/14, D06N 3/18

(54) **FILM ÉLASTIQUE POLYURÉTHANE, RESPIRANT ET IMPERMÉABLE, EN PARTICULIER POUR DES APPLICATIONS DANS LE DOMAINE MÉDICAL, ET PROCÉDÉ POUR FABRIQUER UN TEL FILM**

(30) Priorité: 18.12.2023 FR 2314368
(71) Demandeur: ADHEX TECHNOLOGIES, 21300 Chenôve (FR)
(72) Inventeur: WILLIAMS, Maud, 21000 DIJON (FR); MATRAY, Julie, 21110 FAUVERNEY (FR)
(74) Mandataire: Gevers & Orès

(57) **Abrégé**

- Film élastique, respirant et imperméable, en particulier pour des applications dans le domaine médical, et procédé pour fabriquer un tel film.
- Le procédé de fabrication d'un film (1) élastique, respirant et imperméable comporte une étape d'enduction principale, mise en oeuvre par un dispositif d'enduction, consistant à enduire une face (2A) d'un non-tissé (2) réalisé en polyuréthane d'une formulation polyuréthane (4) en phase aqueuse, avec une adaptation d'au moins une caractéristique du non-tissé (2) et/ou d'au moins une caractéristique de la formulation polyuréthane (4), de manière à permettre à la formulation polyuréthane (4), tout en formant une couche (3) en polyuréthane sur ladite face (2A) du non-tissé (2), de pénétrer partiellement dans le non-tissé (2) sans le traverser complètement, ce qui permet d'obtenir un film (1) présentant des propriétés avantageuses en termes de manipulabilité, de porosité, de respirabilité et d'étanchéité à l'eau.

## Description

### Domaine technique

La présente invention concerne un film élastique, respirant et imperméable, en particulier pour des applications dans le domaine médical, ainsi qu'un procédé pour fabriquer un tel film.

### État de la technique

Bien que non exclusivement, le film tel que considéré dans la présente invention peut être employé plus particulièrement comme support dans diverses applications médicales, telles que des pansements pour le soin d'une plaie ou des patchs de recouvrement par exemple. On peut également citer des applications pour des complexes avec des formulations très fluides, peu collantes de type silicone, qui nécessitent une enduction en direct sur la face film du complexe.

Pour pouvoir être utilisé dans de telles applications, ce film doit présenter diverses propriétés et notamment être élastique, respirant (à la vapeur d'eau) et imperméable (à l'eau notamment).

Aussi, il existe un intérêt et un besoin de réaliser un film présentant de telles propriétés.

Par ailleurs, on connaît, par le document CN203651110U, un film pour un traitement médical et notamment pour des pansements. Ce film comprend un non-tissé en polypropylène pourvu sur une face d'une couche en polyuréthane, qui pénètre partiellement dans le non-tissé. Le résultat principal recherché pour ce film est d'obtenir une liaison forte entre le non-tissé et la couche en polyuréthane.

Or, même si le document CN203651110U mentionne également que le film obtenu est respirant, il peut être utile d'augmenter encore la respirabilité de ce dernier, en particulier pour son utilisation dans les applications envisagées dans la présente demande de brevet où la respirabilité est souvent un critère important. A titre d'illustration, il est nécessaire, par exemple dans l'application à un pansement pour le soin d'une plaie, de disposer d'une respirabilité élevée, pour permettre le cas échéant à un excès d'humidité de s'évaporer.

Aussi, il existe un intérêt et un besoin de réaliser un film présentant les propriétés précitées avec notamment une respirabilité élevée (à la vapeur d'eau).

### Exposé de l'invention

La présente invention a pour objet de proposer un film élastique, respirant et imperméable, susceptible d'être utilisé notamment dans les applications précitées et de répondre à ce besoin. Elle concerne un film comprenant un non-tissé pourvu sur une face d'une couche en polyuréthane, dont la matière pénètre partiellement dans ledit non-tissé sans le traverser complètement.

Selon l'invention, le non-tissé est réalisé en polyuréthane et la couche en polyuréthane est obtenue à partir d'une formulation polyuréthane en phase aqueuse (sans solvant).

Ainsi, grâce à l'invention, on obtient un film qui présente des caractéristiques avantageuses en termes de manipulabilité, de porosité, d'étanchéité et de respirabilité à l'eau, comme précisé ci-dessous. En particulier, ce film présente des propriétés mécaniques uniques, en combinant à la fois élasticité, respirabilité à la vapeur d'eau, et imperméabilité aux liquides et en particulier à l'eau. Plus précisément :
- il est élastique, grâce notamment à l'équilibre entre les caractéristiques de la couche en polyuréthane et du non-tissé, ainsi qu'à la présence partielle du polyuréthane dans l'épaisseur du non-tissé ;
- il est respirant, grâce notamment à la nature chimique du polymère polyuréthane, à l'épaisseur de la couche (en polyuréthane) déposée et à l'utilisation d'un non-tissé en polyuréthane ;
- il est imperméable, grâce notamment à la couche - continue - en polyuréthane présente en surface d'une des faces du non-tissé ; et
- il est manipulable pour pouvoir être appliqué et retiré facilement sans rupture, grâce notamment à sa caractéristique de module d'Young.

Grâce à ses propriétés avantageuses, ce film est particulièrement bien adapté à une utilisation dans le domaine médical, notamment pour des pansements pour le soin de plaies, pour des patchs de recouvrement, ou pour l'utilisation en tant que support pour des formulations à faible collant de type silicone.

Par rapport, au produit du document CN203651110U, le film de la présente invention comporte, notamment, un non-tissé qui est réalisé en polyuréthane, ce qui permet d'augmenter la respirabilité du film par rapport à un produit avec un non-tissé en polypropylène. De plus, la couche en polyuréthane du film est obtenue à partir d'une formulation polyuréthane en phase aqueuse (c'est-à-dire sans solvant), ce qui permet de ne pas endommager le non-tissé en polyuréthane comme cela aurait été le cas avec l'application d'une formulation polyuréthane en phase solvant sur un non-tissé en polyuréthane.

Pour obtenir un film avec une solution de polyuréthane qui pénètre partiellement dans le non-tissé sans le traverser complètement, avantageusement, le non-tissé présente des valeurs appropriées d'épaisseur, de grammage, de caractère hydrophobe et/ou de perméabilité, telles que précisées ci-dessous. En outre, en complément ou en variante, pour obtenir cette pénétration partielle de la solution polyuréthane, de façon avantageuse, la formulation polyuréthane utilisée présente des valeurs appropriées de viscosité et/ou d'extrait sec, telles que précisées également ci-dessous.

Dans un mode de réalisation préféré, le film (élastique, respirant et imperméable) présente, au moins certaines des caractéristiques suivantes :
- un grammage, mesuré selon la méthode de mesure de la norme FTM12, compris entre 20 et 300 g/m² ;
- une résistance à la rupture, mesurée selon la méthode de mesure de la norme NF EN 29073-3, supérieure à 300 N/m en direction longitudinale de la machine et supérieure à 200 N/m en direction transversale de la machine ;
- un allongement à la rupture, mesuré selon la méthode de mesure de la norme NF EN 29073-3, supérieur à 2 % en direction longitudinale de la machine et supérieur à 2 % en direction transversale de la machine ;
- un module de Young en direction transversale de la machine, mesuré selon la méthode de mesure de la norme NF EN 29073-3, compris entre 5 et 250 MPa ; et
- un taux de transmission de vapeur d'eau, mesuré selon la méthode de mesure de la norme NF EN 13726-2, supérieur à 1000 g/m²/24h.

Ces caractéristiques permettent au film d'obtenir les propriétés précitées.

Avantageusement, la couche en polyuréthane est obtenue à partir d'une formulation polyuréthane comportant un polymère polyuréthane aliphatique base polyéther/polyester ou polyéther seul. De préférence, la formulation polyuréthane (en phase aqueuse) comporte également un réticulant isocyanate, ce qui permet de renforcer les propriétés mécaniques et la durabilité du film formé à partir d'une telle formulation en phase aqueuse. Avantageusement, le réticulant est un prépolymère diisocyanate aliphatique base hexaméthylène.

En outre, avantageusement, le film comprend également au moins une couche d'adhésif appliquée sur au moins l'une des faces de l'ensemble formé du non-tissé et de la couche en polyuréthane.

La présente invention concerne également un procédé de fabrication d'un film élastique, respirant et imperméable.

Selon l'invention, ledit procédé comprend au moins une étape d'enduction principale, mise en oeuvre par un dispositif d'enduction, consistant à enduire une face d'un non-tissé réalisé en polyuréthane d'une formulation polyuréthane en phase aqueuse, avec une adaptation d'au moins une caractéristique de la formulation polyuréthane et/ou d'au moins une caractéristique du non-tissé, de manière à permettre à la formulation polyuréthane, tout en formant une couche de polyuréthane sur ladite face du non-tissé, de pénétrer partiellement dans le non-tissé sans le traverser complètement.

Ainsi, grâce à l'utilisation d'un non-tissé et d'une formulation polyuréthane dont une ou plusieurs caractéristiques ont été choisies de façon appropriée, le procédé est en mesure de réaliser une enduction permettant à la formulation polyuréthane de pénétrer partiellement dans le non-tissé sans le traverser complètement, ce qui permet d'obtenir un film présentant les propriétés recherchées, à savoir un film qui est notamment élastique, respirant et imperméable à l'eau.

Avantageusement, la ou les caractéristiques du non-tissé qui sont adaptées comprennent au moins l'une des caractéristiques suivantes du non-tissé, associées aux domaines de valeurs correspondants :
- une épaisseur, mesurée selon la méthode de mesure de la norme NF EN ISO 534, comprise entre 0,09 et 0,18 mm ;
- un grammage, mesuré selon la méthode de mesure FTM12, compris entre 20 et 300 g/m², et de préférence entre 30 et 50 g/m² ;
- une perméabilité à l'air, mesurée selon la méthode de mesure WSP 70.1, comprise entre 50 et 2000 L/m²/s ; et
- un taux de transmission de vapeur d'eau, mesuré selon la méthode de mesure de la norme NF EN 13726-2, supérieur à 3000 g/m²/24h.

En outre, de façon avantageuse, la ou les caractéristiques de la formulation polyuréthane qui sont adaptées comprennent au moins l'une des caractéristiques suivantes de la formulation polyuréthane, associées aux domaines de valeurs correspondants :
- une viscosité, mesurée à 25°C, vitesse 2 selon la méthode de mesure de la norme NF EN ISO 2555, comprise entre 1000 et 2000 mPa.s ; et
- un extrait sec compris entre 58 et 62 %.

Dans un mode de réalisation préféré, pour obtenir la couche en polyuréthane, le procédé utilise une dispersion de polymère polyuréthane aliphatique à base de polyéther/polyester ou polyéther seul, combinée à un réticulant diisocyanate aliphatique à base d'hexaméthylène.

Dans un mode de réalisation, le procédé comprend également une étape d'enduction auxiliaire, mise en oeuvre après l'étape d'enduction principale, consistant à déposer au moins une couche d'adhésif sur au moins l'une des faces de l'ensemble formé du non-tissé et de la couche en polyuréthane.

De préférence, le dispositif d'enduction est configuré pour mettre en oeuvre au moins l'étape d'enduction principale par l'intermédiaire de l'un des types d'enduction suivants :
- une enduction par gravure ;
- une enduction à applicateur spiral ;
- une enduction par rouleau de transfert ;
- une enduction par buse à filière plate.

### Brève description des figures

D'autres avantages et caractéristiques ressortiront mieux de la description qui va suivre de plusieurs modes de réalisation, donnés à titre d'exemples non limitatifs, d'un film conforme à l'invention et de son procédé de fabrication, en se référant notamment aux figures annexées. Sur ces figures, des références identiques désignent des éléments semblables.
La figure 1 est une vue schématique partielle, en coupe, d'un film élastique, respirant et imperméable, conforme à un mode de réalisation de l'invention.
La figure 2 est une vue schématique d'un premier mode de réalisation d'un dispositif d'enduction faisant partie d'un dispositif de fabrication d'un film élastique, respirant et imperméable.
La figure 3 est une vue schématique d'un deuxième mode de réalisation d'un dispositif d'enduction faisant partie d'un dispositif de fabrication d'un film élastique, respirant et imperméable.
La figure 4 est une vue schématique d'un troisième mode de réalisation d'un dispositif d'enduction faisant partie d'un dispositif de fabrication d'un film élastique, respirant et imperméable.
La figure 5 est une vue schématique d'un quatrième mode de réalisation d'un dispositif d'enduction faisant partie d'un dispositif de fabrication d'un film élastique, respirant et imperméable.

### Description détaillée

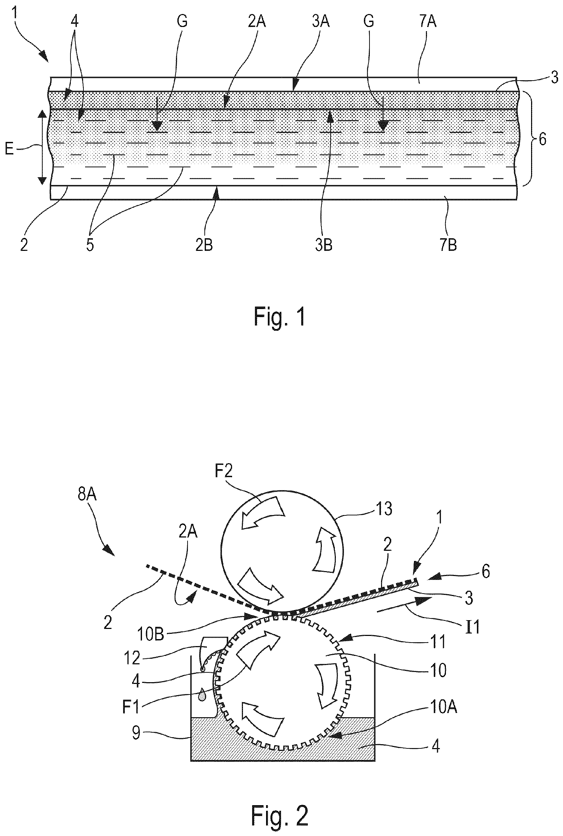

Le film 1 permettant d'illustrer l'invention et représenté partiellement selon un mode de réalisation particulier sur la figure 1 est un film élastique, respirant et imperméable, comme précisé ci-dessous.

Dans le mode de réalisation représenté sur la figure 1, le film 1 comprend un non-tissé 2 pourvu sur l'une 2A de ses faces 2A et 2B (opposées), d'une couche 3 en polyuréthane, dont la matière (c'est-à-dire la formulation polyuréthane 4) a pénétré, à partir de cette face 2A dans ledit non-tissé 2. Dans le cadre de la présente invention, une partie de la formulation polyuréthane 4 est intégrée partiellement dans le non-tissé 2 (pourvue de fibres 5 représentées très schématiquement sur la figure 1), sans le traverser complètement.

La formulation polyuréthane 4 est représentée schématiquement par des points noirs sur la figure 1, avec des points devenant plus petits vers l'intérieur de la couche 3 pour illustrer que la concentration de la formulation polyuréthane 4 diminue vers l'intérieur de la couche 3 à partir de la face 2A du non-tissé 2. La couche 3 présente deux faces 3A et 3B (opposées), dont la face 3B est en contact de la face 2A du non-tissé 2.

Dans le cadre de la présente invention, on prévoit une adaptation d'au moins une caractéristique de la formulation polyuréthane 4 utilisée et/ou une adaptation d'au moins une caractéristique du non-tissé 2 utilisé, de manière à permettre à la formulation polyuréthane 4 qui forme la couche 3 de polyuréthane, de pénétrer dans une partie du non-tissé 2 (dans le sens illustré par des flèches G) sans traverser complètement l'épaisseur E du non-tissé 2, comme illustré par les points noirs sur la figure 1.

Le non-tissé 2 présente donc une ou plusieurs caractéristiques particulières, en fonction du mode de réalisation envisagé, qui permettent à la formulation polyuréthane 4 de pénétrer dans celui-ci sans le traverser.

Afin notamment d'obtenir cette pénétration partielle dans le non-tissé 2, au moins certaines (et de préférence l'ensemble) des caractéristiques suivantes sont à prendre en compte pour le choix du non-tissé 2 :
- l'épaisseur E (figure 1) du non-tissé 2 ;
- le grammage du non-tissé 2 ;
- le caractère hydrophobe du non-tissé 2, obtenu par sa formulation chimique et/ou à partir d'un traitement approprié ;
- la perméabilité à l'air du non-tissé 2.

Dans un mode de réalisation préféré, pour que le film 1 obtienne les propriétés recherchées, au moins certaines de ces caractéristiques du non-tissé 2 présentent les valeurs suivantes :
- une épaisseur, mesurée selon la méthode de mesure de la norme NF EN ISO 534, comprise entre 0,09 et 0,18 mm ;
- un grammage, mesuré selon la méthode de mesure de la norme FTM12 (« FINAT Test Method »), compris entre 20 et 300 g/m², et de préférence entre 30 et 50 g/m² ; et
- une perméabilité à l'air, mesurée selon la méthode de mesure WSP 70.1, comprise entre 50 et 2000 L/m²/s.

Dans le cadre de la présente invention, les plages de valeurs présentées sous forme de « entre x et y » incluent les bornes x et y, les entiers compris entre ces bornes, ainsi que tous les autres nombres réels compris entre ces bornes.

De préférence, le non-tissé 2 utilisé présente également ;
- un taux de transmission de vapeur d'eau, mesuré selon la méthode de mesure de la norme NF EN 13726-2, supérieur à 3000 g/m²/24h ;
- une résistance à la rupture (mesurée selon la méthode de mesure de la norme NF EN 29073-3) supérieure à 300 N/m en direction longitudinale de la machine (ci-après MD pour « machine direction » en anglais) et supérieure à 250 N/m en direction transversale de la machine (ci-après CD pour « cross direction » en anglais) ; et
- un allongement à la rupture supérieur à 2 % en direction longitudinale de la machine et supérieur à 7 % en direction transversale de la machine.

Le non-tissé 2 est réalisé en polyuréthane.

Il pourrait être envisagé que le non-tissé 2 soit réalisé en polytéréphtalate d'éthylène (PET).

A titre d'illustration, le tableau T1 ci-dessous permet de mettre en évidence les caractéristiques principales du non-tissé 2 pour cinq non-tissés différents nommés NT1 à NT5, qui sont susceptibles d'être utilisés pour réaliser le film 1 :

| Références commerciales | NT1 : BR7398-050 | NT2 : BR6331-034 | NT3: BR7396-045 | NT4 : BR7378-040 | NT5 : 1E M045A75T |
|---|---|---|---|---|---|
| Technologie de fabrication | « Wetlaid » | « Wetlaid » | « Wetlaid » | « Wetlaid » | « Meltblown » |
| Nature des fibres | Cellulose / PET | Cellulose / PET | Cellulose / PET | Cellulose/ PET | TPU |
| Traitement | Fluorocarboné | Aucun | Traitement non fluoré | Traitement non fluoré | Aucun |
| Grammage (*g*/*m²)* | 45 | 34 | 45 | 40 | 45 |
| Epaisseur *(µm)* | 124 | 95 | 108 | 108 | 170 |
| Perméabilité *(L*/*m²*/*s)* | 320 | 1445 | 98 | 650 | 750 |
| Essai de la goutte d'eau (*s)* | 999 | >999 | - | 999 | - |
| Essai de la goutte d'alcool *(s)* | >540 | 354 | 540 | >540 | - |
| Résistance à la rupture *(N*/*m)* | MD : 1960 | MD : 774 | MD : 2795 | MD : 1450 | MD : 440 |
| | CD : 1100 | CD : 619 | CD : 755 | CD : 820 | CD : 300 |
| Allongement à la rupture *(%)* | MD : 5.3 | MD : 18.5 | MD : 2.9 | MD: 6.0 | MD : 230 |
| | CD : 12.9 | CD : 24.7 | CD : 9.8 | CD : 13.0 | CD : 220 |
| Respirabilité à la vapeur d'eau *(g*/*m²*/*24h)* | 5330 | 5425 | 4715 | 5395 | 6030 |

Ce tableau T1 présente pour les cinq exemples de non-tissé NT1 à NT5 considérés :
- leur référence commerciale ;
- la technologie de fabrication utilisée ;
- la nature des fibres du non-tissé ;
- un éventuel traitement ;
- le grammage en g/m² (mesuré selon la méthode de mesure de la norme FTM12 (pour « FINAT Test Method »)) ;
- l'épaisseur en µm (mesurée selon la méthode de mesure de la norme NF EN ISO 534) ;
- la perméabilité à l'air en L/m²/s (mesurée selon la méthode de mesure WSP 70.1) ;
- un essai de la goutte d'eau (« Water drop test » en anglais) en secondes ;
- un essai de la goutte d'alcool (« Alcohol drop test ») en secondes ;
- la résistance à la rupture en N/m (mesurée selon la méthode de mesure de la norme NF EN 29073-3) ;
- l'allongement à la rupture en % (mesurée selon la méthode de mesure de la norme NF EN 29073-3) ; et
- le taux de transmission à la vapeur d'eau en g/m²/24h (mesurée selon la méthode de mesure de la norme NF EN 13726-2).

Pour ces cinq exemples de non-tissé NT1 à NT5, les valeurs de grammage, d'épaisseur, de résistance à la rupture et d'allongement à la rupture sont comprises dans les domaines de valeurs recherchées pour le non-tissé 2, tels qu'indiqués ci-dessus.

Par ailleurs, en complément ou en variante des caractéristiques particulières précitées du non-tissé 2, afin de permettre à la formulation polyuréthane 4 de pénétrer dans le non-tissé 2, sans le traverser complètement, au moins l'une des caractéristiques suivantes est à prendre en compte pour le choix de la formulation polyuréthane 4 :
- sa viscosité ; et
- son extrait sec.

L'ajustement de la viscosité permet d'éviter la traversée lors de l'enduction, tout en maîtrisant le grammage déposé.

Dans un mode de réalisation préféré, pour obtenir l'objectif recherché précité, la formulation polyuréthane 4 présente au moins l'une des caractéristiques suivantes :
- une viscosité (mesurée à 25°C, vitesse 2 selon la méthode de mesure de la norme NF EN ISO 2555) comprise entre 1000 et 2000 mPa.s ; et
- un extrait sec compris entre 58 et 62 %.

La formulation polyuréthane 4 est une dispersion en phase aqueuse, sans solvant. Elle ne contient donc pas de solvant susceptible d'endommager le non-tissé 2 en polyuréthane.

De plus, de préférence, la formulation polyuréthane 4 (en phase aqueuse) comporte un réticulant qui permet de renforcer les propriétés mécaniques du film 1 obtenu.

Ainsi, dans un mode de réalisation préféré, la formulation polyuréthane (nommée UE-12) susceptible d'être utilisée, comprend les composants suivants :
- un polymère polyuréthane aliphatique base polyéther/polyester, pour 100 parts ; et
- un prépolymère diisocyanate aliphatique base hexaméthylène (HDI), pour 3 parts (réticulant).

L'utilisation du réticulant entraîne une durée de vie du mélange, de l'ordre de 6 heures.

Par ailleurs, le film 1 peut également comprendre au moins une couche d'adhésif.

Dans un premier mode de réalisation, le film 1 comprend deux couches d'adhésif 7A et 7B appliquées, respectivement, sur la face 3A de la couche 3 en polyuréthane et sur la face 2B du non-tissé 2, comme représenté sur la figure 1.

Dans un second mode de réalisation, le film 1 comprend une seule couche d'adhésif appliquée sur l'une des faces de l'ensemble 6 formé du non-tissé 2 et de la couche 3 en polyuréthane. Dans ce premier mode de réalisation, la couche d'adhésif peut être appliquée sur la face 2B du non-tissé 2, comme la couche d'adhésif 7B de la figure 1 ou sur la face 3A de la couche 3 en polyuréthane, comme la couche d'adhésif 7A de la figure 1.

Le film 1 adhésif ainsi obtenu peut être collé facilement, et ceci par l'une de ses faces ou par ses deux faces selon le mode de réalisation utilisé, notamment en fonction de l'application envisagée.

Le film 1, tel que décrit ci-dessus, est obtenu à l'aide d'un procédé de fabrication précisé ci-dessous. Ce procédé de fabrication comprend, notamment, une étape d'enduction principale particulière.

Cette étape d'enduction principale qui est mise en oeuvre par un dispositif d'enduction, consiste à enduire une face 2A d'un non-tissé 2 d'une formulation polyuréthane 4, avec une adaptation d'au moins une caractéristique de la formulation polyuréthane 4 et/ou d'au moins une caractéristique du non-tissé 2, de manière à permettre à la formulation polyuréthane 4, tout en formant une couche 3 de polyuréthane sur ladite face 2A du non-tissé 2, de pénétrer partiellement dans le non-tissé 2 (dans le sens illustré par les flèches G sur la figure 1), sans le traverser complètement.

La formulation polyuréthane 4 permettant d'obtenir la couche 3 correspond à une dispersion en phase aqueuse de polymère polyuréthane aliphatique à base de polyéther/polyester, combinée à un réticulant isocyanate aliphatique de type HDI (diisocyanate d'hexaméthylène).

Le procédé permet donc de réaliser un complexe (film 1) comprenant un non-tissé 2, notamment en polyuréthane, recouvert d'une couche 3 en polyuréthane. Le non-tissé 2 peut être de couleur variable (blanc, chair, brun...) en fonction de l'application visée.

Dans le cadre de la présente invention, le procédé, et notamment son étape d'enduction, peuvent être mis en oeuvre à partir de différents dispositifs d'enduction tels que, par exemple, ceux décrits ci-dessous, à titre d'illustration, en référence aux figures 2 à 5.

Dans un premier mode de réalisation représenté sur la figure 2, le procédé de fabrication utilise un dispositif d'enduction 8A configuré pour mettre en oeuvre une enduction par gravure (ou « gravure coating » en anglais).

Dans ce premier mode de réalisation, le dispositif d'enduction 8A comprend un réservoir 9 contenant la formulation polyuréthane 4 et un cylindre (ou rouleau) gravé 10 rotatif, pourvu d'une surface externe 11 gravée (avec des aspérités et/ou des évidements). Le cylindre gravé 10 est agencé de sorte qu'une partie inférieure 10A dudit cylindre gravé 10 est plongée dans la formulation polyuréthane 4.

En tournant, dans le sens illustré par des flèches F1, le cylindre gravé 10 emporte avec sa surface externe 11 gravée de la formulation polyuréthane 4. L'excédent (ou surplus) de formulation polyuréthane 4 est enlevé au passage et au contact d'un racloir 12.

De plus, le non-tissé 2 à enduire de la formulation polyuréthane 4 est pressée par un cylindre (ou rouleau) de pression 13 rotatif contre une partie supérieure 10B du cylindre gravé 10. Ce cylindre de pression 13 tourne dans le sens illustré par des flèches F2, à savoir en sens inverse au sens F1 de rotation du cylindre gravé 10. En tournant dans les sens, respectivement, des flèches F1 et F2, le cylindre gravé 10 et le cylindre de pression 13 participent au déplacement non-tissé 2 dans le sens illustré par une flèche I1 sur la figure 2. Pendant ce déplacement, la formulation polyuréthane 4 qui est amenée du réservoir 9 par le cylindre gravé 10 est déposée sur la face 2A du non-tissé 2, arrivant en contact du cylindre gravé 10, à une zone de contact (au niveau de la partie supérieure 10B). La formulation polyuréthane 4 pénètre alors en partie dans le non-tissé 2 sans le traverser.

En aval de cette zone de contact, le non-tissé 2 est donc enduit de la formulation polyuréthane 4, ce qui permet d'obtenir le film 1.

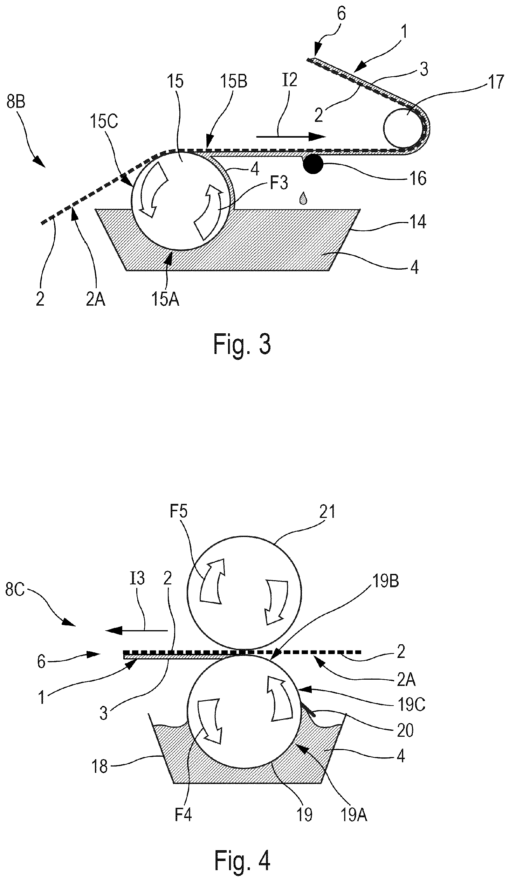

Dans un deuxième mode de réalisation représenté sur la figure 3, le procédé de fabrication utilise un dispositif d'enduction 8B configuré pour mettre en oeuvre une enduction à applicateur spiral (ou « Meyer bar coating » en anglais).

Dans ce deuxième mode de réalisation, le dispositif d'enduction 8B comprend un réservoir 14 contenant la formulation polyuréthane 4 et un cylindre (ou rouleau) applicateur 15 rotatif, pourvu d'une surface externe 15C non lisse. Le cylindre applicateur 15 est agencé de sorte qu'une partie inférieure 15A dudit cylindre applicateur 15 est plongée dans la formulation polyuréthane 4.

En tournant, dans le sens illustré par des flèches F3, le cylindre applicateur 15 emporte avec sa surface externe 15C de la formulation polyuréthane 4 et la dépose sur la face 2A du non-tissé 2, en contact d'une partie supérieure 15B du cylindre applicateur 15, à une zone de contact. En aval de cette zone de contact, dans le sens de déplacement I2 du non-tissé 2, est agencé un applicateur spiral 16 (« Meyer Bar ») qui enlève l'excédent de formulation polyuréthane 4 pour obtenir l'épaisseur souhaitée de la couche 3 de polyuréthane. La formulation polyuréthane 4 pénètre alors en partie dans le non-tissé 2 sans le traverser.

En aval de l'applicateur spiral 16, le non-tissé 2 est donc enduit de la formulation polyuréthane 4, ce qui permet d'obtenir le film 1. Dans le mode de réalisation représenté sur la figure 3, le dispositif d'enduction 8B comporte également un rouleau auxiliaire 17 pour amener le film 1 dans une direction souhaitée.

Par ailleurs, dans un troisième mode de réalisation représenté sur la figure 4, le procédé de fabrication utilise un dispositif d'enduction 8C configuré pour mettre en oeuvre une enduction par rouleau de transfert (ou « kiss coating » en anglais).

Dans ce troisième mode de réalisation, le dispositif d'enduction 8C comprend un réservoir 18 contenant la formulation polyuréthane 4 et un cylindre (ou rouleau) applicateur 19 rotatif, pourvu d'une surface externe 19C non lisse. Le cylindre applicateur 19 est agencé de sorte qu'une partie inférieure 19A dudit cylindre applicateur 19 est plongée dans la formulation polyuréthane 4.

En tournant, dans le sens illustré par des flèches F4, le cylindre applicateur 19 emporte avec sa surface externe 19C de la formulation polyuréthane 4 afin de la déposer sur la face 2A du non-tissé 2, en contact d'une partie supérieure 19B du cylindre applicateur 19, à une zone de contact. À la sortie du cylindre applicateur 19 du réservoir 18, est agencée une lame 20 qui racle le surplus de formulation polyuréthane 4.

De plus, le non-tissé 2 à enduire de la formulation polyuréthane 4 est pressée par un cylindre (ou rouleau) de pression 21 rotatif, contre la partie supérieure 19B du cylindre applicateur 19. Ce cylindre de pression 21 tourne dans le sens illustré par des flèches F5, à savoir en sens inverse à celui (flèches F4) du cylindre applicateur 19. En tournant dans les sens, respectivement, des flèches F4 et F5, le cylindre applicateur 19 et le cylindre de pression 21 participent au déplacement non-tissé 2 dans le sens illustré par une flèche I3 sur la figure 4. Pendant ce déplacement, la formulation polyuréthane 4 est déposée sur la face 2A du non-tissé 2, arrivant en contact du cylindre applicateur 19, à une zone de contact (au niveau de la partie supérieure 19B). La formulation polyuréthane 4 pénètre alors en partie dans le non-tissé 2 sans le traverser.

En aval de cette zone de contact, le non-tissé 2 est donc enduit de la formulation polyuréthane 4, ce qui permet d'obtenir le film 1.

La quantité d'enduit déposée (à savoir la formulation polyuréthane 4 déposée) dépend notamment de la vitesse d'enduction, de la vitesse de rotation du cylindre applicateur 19, de la pression de contact générée par le cylindre de pression 21, de la viscosité de la formulation polyuréthane 4 et de caractéristiques de la lame 20.

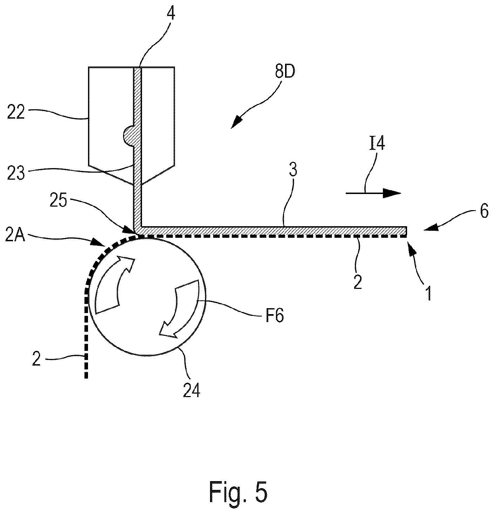

Dans un quatrième mode de réalisation représenté sur la figure 5, le procédé de fabrication utilise un dispositif d'enduction 8D configuré pour mettre en oeuvre une enduction par buse à filière plate (ou « slot die coating » en anglais).

Dans ce quatrième mode de réalisation, le dispositif d'enduction 8D comprend une filière 22 pourvue d'une fente 23, configurée pour distribuer la formulation polyuréthane 4.

Le dispositif d'enduction 8D comprend également un cylindre 24 destiné au déplacement du non-tissé 2. Ce cylindre 24 tourne dans le sens illustré par des flèches F6, pour participer au déplacement non-tissé 2 dans le sens montré par une flèche I4. Le non-tissé 2 est déplacé sous la filière 22. La formulation polyuréthane 4 est déposée sur la face 2A du non-tissé 2, au niveau d'une zone d'enduction 25 située directement sous la filière 22. La formulation polyuréthane 4 pénètre alors en partie dans le non-tissé 2 sans le traverser.

En aval de cette zone d'enduction, le non-tissé 2 est donc enduit de la formulation polyuréthane 4, ce qui permet d'obtenir le film 1.

Le dispositif d'enduction 8D permet, notamment, de bien maîtriser l'épaisseur de la formulation polyuréthane 4 déposée.

Les dispositifs d'enduction 8A, 8B, 8C et 8D décrits ci-dessus permettent de réaliser une enduction, en grande laize, d'une fine couche 3 d'une formulation polyuréthane 4 sur un non-tissé 2, sans provoquer la traversée de la formulation au coeur des fibres 5 du non-tissé 2.

Quel que soit le dispositif d'enduction 8A, 8B, 8C et 8D utilisé, le procédé de fabrication prend en compte les caractéristiques (telles que l'épaisseur, le grammage, le caractère hydrophobe, la perméabilité) précisées ci-dessus pour le non-tissé 2 et les caractéristiques précisées ci-dessus (telles que la viscosité et l'extrait sec) pour la formulation polyuréthane 4.

Dans un mode de réalisation préféré, pour obtenir la couche 3 en polyuréthane, le procédé utilise, comme formulation polyuréthane 4, une dispersion de polymère polyuréthane aliphatique à base de polyéther/polyester 4, combinée à un réticulant isocyanate aliphatique de type HDI (diisocyanate d'hexaméthylène).

Les caractéristiques (viscosité, rhéologie) de la formulation polyuréthane 4 (en phase aqueuse) sans solvant agressif permettent de fabriquer un complexe (film 1) en ligne sans interface entre le non tissé 2 et la couche 3. Ce complexe présente ainsi des caractéristiques intéressantes en termes de manipulabilité, de porosité, de respirabilité et d'étanchéité à l'eau.

De plus, le procédé de fabrication utilise un non-tissé 2 en polyuréthane.

En outre, dans un mode de réalisation particulier, le procédé de fabrication comprend, de plus, une étape d'enduction auxiliaire mise en oeuvre par un dispositif d'enduction auxiliaire usuel, après l'étape d'enduction principale précisée ci-dessus. Le dispositif d'enduction auxiliaire (non représenté) applique, de façon usuelle, sur au moins l'une des faces 2B et 3A de l'ensemble 6 (figure 1) une couche d'adhésif, telle que la couche d'adhésif 7A ou la couche d'adhésif 7B de la figure 1. Le film 1 adhésif ainsi obtenu peut alors être collé facilement sur un support de l'application envisagée.

Le tableau T2 ci-dessous permet de mettre en évidence les caractéristiques principales du film 1, obtenu de préférence à partir de l'un des procédés de fabrication et d'enduction précités, pour quatre exemples 1A, 1B, 1C et 1D de film.

| Film | 1A | 1B | 1C | 1D |
|---|---|---|---|---|
| Construction | UE-12 sur NT5 | UE-12 sur NT1 | UE-12 sur NT3 | UE-12 sur NT4 |
| Grammage de formulation polyuréthane déposée *(g*/*m²)* | 30 | 25 | 25 | 25 |
| Résistance à la rupture *(N*/*cm)* | MD : 4.7 -10.7 | MD : 18.4 | MD : 29.9 | MD : 13.7 |
| | CD : 2.4 - 8.4 | CD : 13.0 | CD : 8.5 | CD : 95 |
| Allongement à la rupture *(%)* | MD : 220-360 | MD : 6.2 | MD : 3.2 | MD : 7.3 |
| | CD : 170-310 | CD : 13.4 | CD : 12.0 | CD : 16.3 |
| Module d'Young en sens transversal (M Pa) | 11.4 | 243 | 203 | 163 |
| MVTR *(g*/*m²*/*24h)* | >1400 | >2000 | >1800 | >1800 |

Ce tableau T2 présente pour les quatre exemples 1A, 1B, 1C et 1D considérés de film 1 :
- leur construction, à savoir le non-tissé utilisé (en l'occurrence les non-tissés NT1 à NT5 précisés ci-dessus) associé à la même formulation polyuréthane (UE-12) ;
- le grammage de la formulation polyuréthane déposée en g/m² (mesuré selon la méthode de mesure de la norme FTM12 (« FINAT Test Method »)) ;
- la résistance à la rupture en N/m (mesurée selon la méthode de mesure de la norme NF EN 29073-3) ;
- l'allongement à la rupture en % (mesuré selon la méthode de mesure de la norme NF EN 29073-3) ;
- le module d'Young en MPa (mesuré selon la méthode de mesure de la norme NF EN 29073-3) ;
- le taux de transmission de vapeur d'eau (MVTR pour « Moisture Vapour Transmission Rate » en anglais), à savoir le débit de perméation de l'eau à travers une membrane, mesuré (selon la méthode de mesure de la norme NF EN 13726-2) en masse par unité de surface par unité de temps, en l'occurrence en g/m²/24h.

Ce tableau T2 permet de mettre en évidence les caractéristiques avantageuses suivantes recherchées pour le film 1, qui sont présentes dans chacun des quatre exemples 1A, 1B, 1C et 1D considérés :
- un grammage compris entre 20 et 300 g/m² ;
- une résistance à la rupture supérieure à 300 N/m en direction longitudinale de la machine et supérieure à 200 N/m en direction transversale de la machine ;
- un allongement à la rupture supérieur à 2 % en direction longitudinale de la machine et supérieur à 2 % en direction transversale de la machine ;
- un module d'Young (ou module d'élasticité en sens transversal) compris entre 5 et 250 MPa ; et
- un taux de transmission de vapeur d'eau supérieur à 1000 g/m²/24h.

Le film 1 présente également des propriétés avantageuses en termes de brillance.

La brillance est mesurée à l'aide d'un brillancemètre qui, de façon usuelle, projette un faisceau lumineux sur la surface du film selon un angle d'incidence présentant une valeur d'angle donnée par rapport à la normale à la surface du film, généralement à 20°, 60° et 85° (par rapport à cette normale), et mesure la quantité de lumière réfléchie selon un angle de réflexion (présentant une même valeur d'angle par rapport à la normale), les angles d'incidence et de réflexion étant symétriques par rapport à la normale.

L'échelle de mesure du brillancemètre est l'unité de brillant (UB), qui est établie à partir d'un étalon de référence en verre noir hautement poli, d'indice de réfraction défini et de réflectance spéculaire de 100 UB à un angle donné. Cette référence est utilisée pour établir un point maximal de calibrage de 100, avec le point minimal établi à 0 pour une surface parfaitement mate.

Le tableau T3 ci-dessous fournit des valeurs de la brillance du film 1, mesurées sur la face externe de la couche 3 en polyuréthane (à savoir la face 3A de la couche 3 du mode de réalisation de la figure 1) du film 1.

| Film | Brillance mesurée à un angle de 60° (UB) | Brillance mesurée à un angle de 85° (UB) |
|---|---|---|
| Différentes mesures | 7.1 | 2.4 |
| | 8.4 | 2.4 |
| | 9.2 | 2.2 |
| | 7.2 | 2.8 |
| | 7.9 | 25 |
| Moyenne des mesures | 8.0 | 25 |

Ce tableau T3 présente pour deux ensembles de mesures réalisées à deux angles de mesure différents, à savoir à 60° et à 85° :
- des valeurs de brillance, exprimées en UB ; et
- la moyenne de ces valeurs, également exprimée en UB.

On peut déduire du tableau T3 que la brillance est faible sur la face 3A de la couche 3 de polyuréthane, apportant ainsi des propriétés avantageuses en termes d'accroche. Cette caractéristique permet ainsi d'augmenter l'affinité et la mouillabilité de la couche adhésive, qui sera appliquée ultérieurement sur cette face 3A de la couche polyuréthane.

Par ailleurs, avec de telles valeurs de brillance, les reflets de la lumière sont réduits, ce qui peut être utile dans des applications dans des environnements lumineux ou sous un éclairage artificiel intense, tels que ceux rencontrés dans un bloc opératoire.

Le film 1 présente également des propriétés avantageuses en termes de rugosité.

La rugosité est mesurée à l'aide d'un rugosimètre digital selon la norme EN ISO 4287.

Le tableau T4 ci-dessous fournit des valeurs de la rugosité du film 1, mesurées respectivement sur la face externe de la couche 3 de polyuréthane (à savoir la face 3A de la couche 3 du mode de réalisation de la figure 1) et sur la face externe du non-tissé 2 (à savoir la face 2B du mode de réalisation de la figure 1) du film 1.

| Film | Ra (µm) | Rz (µm) | Rt (µm) |
|---|---|---|---|
| *Mesures sur la face externe du non-tissé* | 15.780 | 86.911 | 109.976 |
| | 14.442 | 87.713 | 115.593 |
| | 17.703 | 96.509 | 127.714 |
| | 15.647 | 100.626 | 139.315 |
| | 12.607 | 81.859 | 101.403 |
| *Moyenne des mesures sur la face externe du non-tissé* | 15.2 | 90.7 | 118.8 |
| *Mesures sur la face externe de la couche en polyuréthane* | 7.519 | 43.757 | 60.371 |
| | 8.012 | 44639 | 51.721 |
| | 12.153 | 87474 | 150.025 |
| | 10.547 | 67355 | 113.996 |
| | 10.628 | 64131 | 109.864 |
| *Moyenne des mesures sur la face externe de la couche en polyuréthane* | 98 | 61.5 | 97.2 |

Ce tableau T4 présente, dans trois colonnes différentes, respectivement les valeurs suivantes :
- la moyenne Ra qui est la moyenne arithmétique des valeurs absolues des déviations du profil d'évaluation à partir d'une ligne dite moyenne (qui est une ligne de référence sur le profil de la surface, située de manière à équilibrer les hauteurs des aspérités (points hauts) et les profondeurs des creux (points bas)) ;
- la hauteur maximum Rz du profil, représentant la moyenne des sommes des points les plus élevés par rapport à la ligne moyenne et des points les plus bas par rapport à la ligne moyenne ; et
- la rugosité maximum Rt, c'est-à-dire la distance entre le point le plus haut et le point le plus bas.

Ce tableau T4 présente pour chacune de ces trois colonnes :
- plusieurs valeurs de rugosité telles que mesurées, exprimées en µm ; et
- la moyenne de ces valeurs mesurées, également exprimée en µm.

On peut déduire du tableau T4 que, grâce à la couche 3 en polyuréthane, la rugosité du film diminue, provoquant ainsi un aspect lisse à la surface ainsi qu'une augmentation de la disponibilité de la formulation acrylique adhésive, ce qui permet d'obtenir un niveau d'adhésivité constant et élevé.

En revanche, la face du non-tissé présente une rugosité différenciée et accrue (par rapport à la face de la couche en polyuréthane), ce qui permet d'obtenir une surface présentant une sensation tactile particulière, plus agréable au toucher et limitant les frottements. Une telle qualité peut être recherchée dans certaines utilisations telles que les dispositifs médicaux destinés au soin d'une plaie.

Le film 1 présente également des propriétés avantageuses en termes de tension de surface.

La méthode de mesure utilisée pour mettre en évidence cette caractéristique est la méthode de la goutte sessile. Pour ce faire, on dépose une goutte d'eau (déminéralisée) sur la surface de la face considérée du film et on mesure un angle dit de contact qui correspond à l'angle entre la tangente à la courbe de la goutte (au point de contact liquide-solide) et ladite surface.

On sait que l'aptitude d'un liquide à mouiller la surface d'un solide est quantifiée par l'angle de contact formé à l'équilibre à la ligne de jonction de trois phases solide, liquide et vapeur. La forme que prend la goutte sur la surface dépend donc de la tension superficielle du liquide et de la nature de la surface. Un liquide est non mouillant ou mouillant, suivant que son angle de contact sur le solide considéré est supérieur ou inférieur à 90°.

Le tableau T5 ci-dessous fournit des valeurs d'angles de contact du film 1, mesurées sur la face externe de la couche 3 de polyuréthane (à savoir la face 3A de la couche du mode de réalisation de la figure 1) du film 1.

| Film | Angle de contact gauche (°) | Angle de contact droit (°) | Angle de contact moyen (°) |
|---|---|---|---|
| *Mesures sur la face du non-tissé* | 78.0 | 74.4 | 76.2 |
| | 85.0 | 78.6 | 81.8 |
| | 77.0 | 70.3 | 73.7 |
| | 76.4 | 69.3 | 72.8 |
| | 78.4 | 73.9 | 76.2 |
| *Moyenne des mesures sur la face du non-tissé* | 79 | 73 | 76 |

Ce tableau T5 présente, dans trois colonnes différentes, les mesures respectivement :
- d'un angle de contact gauche, mesuré sur le côté gauche de la goutte ;
- d'un angle de contact droit, mesuré sur le côté droit de la goutte ; et
- d'un angle de contact moyen, qui correspond à la moyenne arithmétique des angles de contact gauche et droit.

Ce tableau T5 présente pour chacune de ces trois colonnes :
- plusieurs valeurs telles que mesurées, exprimées en ° ; et
- la moyenne de ces valeurs mesurées, également exprimée en °.

On peut en déduire du tableau T5 que les valeurs d'angle de contact mesurées sont élevées.

Par conséquent, la formulation de la couche en polyuréthane, ainsi que son aspect filmogène, permettent d'apporter des propriétés d'imperméabilité au film 1.

Par ailleurs, le tableau T6 suivant apporte des détails sur des formulations avec différents taux de réticulant isocyanate, dont différentes caractéristiques mesurées sont présentées dans le tableau T7 ci-après.

On prévoit un extrait sec du polymère polyuréthane de 60%.

| Film | Formulation en sec *(part)* | |
|---|---|---|
| **UE-06** | Polymère polyuréthane | 100 |
| | Réticulant isocyanate | 0 |
| **UE-12** | Polymère polyuréthane | 100 |
| | Réticulant isocyanate | 5 |
| **UE-13** | Polymère polyuréthane | 100 |
| | Réticulant isocyanate | 10 |

Il s'avère que la couche en polyuréthane réalisée à partir de la formulation UE-06 (sans isocyanate) se fragilise en contact avec l'eau jusqu'à la formation d'un trou.

L'introduction d'un réticulant isocyanate apporte une résistance à l'eau de la couche en polyuréthane.

Le tableau T7 ci-dessous montre les caractéristiques obtenues sur la couche en polyuréthane avec différents taux d'isocyanates.

| Film | L11420 avec UE-06 | L11421 avec UE-12 | L11422 avec UE-13 |
|---|---|---|---|
| | témoin | 5% de réticulant | 10% de réticulant |
| Grammage *(g*/*m²)* | 27 | 26 | 26 |
| Epaisseur (µm) | 25--30 | 25--30 | 25--30 |
| Décomplexage en T *(cN*/*5cm)* | 5 | 6 | 6 |
| Allongement à la rupture (%) | L : 440 | L : 405 | L : 225 |
| | T : 375 | T : 375 | T : 280 |
| Résistance à la rupture *(N*/*cm)* | L : 4.5 | L : 4.3 | L : 1.6 |
| | T : 2.1 | T : 2.7 | T : 26 |
| Cycle d'hystérésis en traction *(N*/*cm)* | L : 0.3 | L : 0.3 | L : 0.4 |
| | T : 0.2 | T : 0.3 | T : 0.3 |
| Rémanence *(%)* | L : 2.7 | L : 2.5 | L: 3.1 |
| | T : 2.9 | T : 2.7 | T : 3.3 |
| MVTR (contact vapeur) *(g*/*m²*/*24h)* | 1930 | 1730 | 1625 |
| MVTR (contact liquide) *(g*/*m²*/*24h)* | 2140 | 1995 | 3240 |
| Imperméabilité (nombre de trous) | 0 | 0 | 0 |

Ce tableau T7 présente, pour trois couches en polyuréthanes avec différents taux d'isocyanates, les mesures de différents paramètres, déjà présentés ci-dessus à l'exclusion des paramètres suivants :
- le décomplexage en T (en cN/5cm), mesuré selon la méthode de mesure de la norme NF EN ISO 11339 ; et
- la rémanence (en %), mesurée selon la méthode de mesure de la norme NF EN 13726-4.

Il résulte de ce tableau que la couche en polyuréthane (L11421), obtenue avec un taux de 5% de réticulant, présente les meilleures caractéristiques en termes de propriétés mécaniques et de résistance à l'eau.

Les caractéristiques de l'association entre le non-tissé et la formulation de polyuréthane rendent le film 1 souple et manipulable, et notamment suffisamment manipulable pour les applications envisagées.

Par conséquent :
- le film 1 (complexe final) obtenu conserve sa perméabilité à la vapeur d'eau, grâce à l'enduction d'une formulation polyuréthane 4 respirante ; et
- les caractéristiques mécaniques du film 1 obtenues varient en fonction de la nature du non-tissé 2 utilisé. Aussi, un choix approprié du non-tissé 2 permet d'obtenir une ou des caractéristiques mécaniques souhaitées, notamment en fonction de l'application envisagée.

Le film 1, tel que décrit ci-dessus, est particulièrement bien adapté à une utilisation comme pansement ou patch de recouvrement dans le domaine médical. En effet, ce film 1 est :
- élastique et conformable, et ainsi agréable au porté ;
- légèrement embossé en surface de manière à être doux au toucher et limiter les frottements ;
- respirant de manière à permettre le passage de la vapeur d'eau à la surface de la peau et assurer une adhésion parfaite durant toute la période d'utilisation prévue ; et
- étanche à l'eau de manière à augmenter la durée d'application sur la peau.

Le film 1, tel que décrit ci-dessus, peut être utilisé dans de nombreuses autres applications, en particulier (mais non exclusivement) dans le secteur médical. Plus généralement, le film 1 peut être utilisé dans toutes les applications dans lesquelles ses caractéristiques avantageuses (et notamment ses propriétés mécaniques uniques, combinant à la fois élasticité, respirabilité à la vapeur d'eau et imperméabilité à l'eau) sont recherchées.

Il est bien évident que les exemples présentés ci-dessus ne sont que des illustrations particulières, en aucun cas limitatives quant aux domaines d'application de la présente invention. De plus, des caractéristiques de certains de ces exemples différents peuvent être combinées entre elles si cela est approprié, sans sortir du cadre de la présente invention.

## Revendications

1. Film élastique, respirant et imperméable, ledit film (1) comprenant un non-tissé (2) pourvu sur une face (2A) d'une couche (3) en polyuréthane, dont la matière (4) pénètre partiellement dans ledit non-tissé (2) sans le traverser complètement,
**caractérisé en ce que** le non-tissé (2) est réalisé en polyuréthane et la couche (3) en polyuréthane est obtenue à partir d'une formulation polyuréthane (4) en phase aqueuse.

2. Film selon la revendication 1,
**caractérisé en ce que** ledit film (1) présente, au moins certaines des caractéristiques suivantes :
- un grammage, mesuré selon la méthode de mesure de la norme FTM12, compris entre 20 et 300 g/m² ;
- une résistance à la rupture, mesurée selon la méthode de mesure de la norme NF EN 29073-3, supérieure à 300 N/m en direction longitudinale de la machine et supérieure à 200 N/m en direction transversale de la machine ;
- un allongement à la rupture, mesuré selon la méthode de mesure de la norme NF EN 29073-3, supérieur à 2 % en direction longitudinale de la machine et supérieur à 2 % en direction transversale de la machine ;
- un module de Young en direction transversale de la machine, mesuré selon la méthode de mesure de la norme NF EN 29073-3, compris entre 5 et 250 MPa ; et
- un taux de transmission de vapeur d'eau, mesuré selon la méthode de mesure de la norme NF EN 13726-2, supérieur à 1000 g/m²/24h.

3. Film selon l'une des revendications 1 et 2,
**caractérisé en ce que** la couche (3) en polyuréthane est obtenue à partir d'une formulation polyuréthane (4) comportant un polymère polyuréthane aliphatique base polyéther/polyester ou polyéther seul.

4. Film selon l'une quelconque des revendications précédentes,
**caractérisé en ce que** la formulation polyuréthane (4) en phase aqueuse comporte un réticulant isocyanate.

5. Film selon la revendication 4,
**caractérisé en ce que** le réticulant est un prépolymère diisocyanate aliphatique base hexaméthylène.

6. Film selon l'une quelconque des revendications précédentes,
**caractérisé en ce qu'**il comprend au moins une couche d'adhésif (7A, 7B) appliquée sur au moins l'une des faces (2B, 3A) de l'ensemble (6) formé du non-tissé (2) et de la couche (3) en polyuréthane.

7. Procédé de fabrication d'un film élastique, respirant et imperméable,
**caractérisé en ce qu'**il comprend au moins une étape d'enduction principale, mise en oeuvre par un dispositif d'enduction (8A, 8B, 8C, 8D), consistant à enduire une face (2A) d'un non-tissé (2) réalisé en polyuréthane d'une formulation polyuréthane (4) en phase aqueuse , avec une adaptation d'au moins une caractéristique du non-tissé (2) et/ou d'au moins une caractéristique de la formulation polyuréthane (4), de manière à permettre à la formulation polyuréthane (4), tout en formant une couche (3) en polyuréthane sur ladite face (2A) du non-tissé (2), de pénétrer partiellement dans le non-tissé (2) sans le traverser complètement.

8. Procédé selon la revendication 7,
**caractérisé en ce que** la ou les caractéristiques du non-tissé (2) qui sont adaptées comprennent au moins l'une des caractéristiques suivantes du non-tissé (2), associées aux domaines de valeurs correspondants :
- une épaisseur, mesurée selon la méthode de mesure de la norme NF EN ISO 534, comprise entre 0,09 et 0,18 mm ;
- un grammage, mesuré selon la méthode de mesure de la norme FTM12, compris entre 20 et 300 g/m² ;
- une perméabilité à l'air, mesurée selon la méthode de mesure de la norme WSP 70.1, comprise entre 50 et 2000 L/m²/s ; et
- un taux de transmission de vapeur d'eau, mesuré selon la méthode de mesure de la norme NF EN 13726-2, supérieur à 3000 g/m²/24h.

9. Procédé selon l'une des revendications 7 et 8,
**caractérisé en ce que** la ou les caractéristiques de la formulation polyuréthane (4) qui sont adaptées comprennent au moins l'une des caractéristiques suivantes de la formulation polyuréthane (4), associées aux domaines de valeurs correspondants :
- une viscosité, mesurée à 25°C, vitesse 2 selon la méthode de mesure de la norme NF EN ISO 2555, comprise entre 1000 et 2000 mPa.s ; et
- un extrait sec compris entre 58 et 62 %.

10. Procédé selon l'une quelconque des revendications 7 à 9,
**caractérisé en ce qu'**il comprend une étape d'enduction auxiliaire, consistant à déposer au moins une couche d'adhésif (7A, 7B) sur au moins l'une des faces (2B, 3A) de l'ensemble (6) formé du non-tissé (2) et de la couche (3) en polyuréthane.

11. Procédé selon l'une quelconque des revendications 7 à 10,
**caractérisé en ce que** le dispositif d'enduction (8A, 8B, 8C, 8D) est configuré pour mettre en oeuvre au moins l'étape d'enduction principale par l'intermédiaire de l'un des types d'enduction suivants :
- une enduction par gravure ;
- une enduction à applicateur spiral ;
- une enduction par rouleau de transfert ;
- une enduction par buse à filière plate.
